# FASCICULE DE BREVET EUROPEEN

(11) **EP 1 590 077 B1**
(45) Date de publication et mention de la délivrance du brevet: **05.05.2010**
(21) Numéro de dépôt: 04703414.5
(22) Date de dépôt: 20.01.2004
(51) Int. Cl.: B01J 13/02

(54) **SYSTEMES POUR MICROENCAPSULATION ET LEURS APPLICATIONS**
SYSTEM ZUR MIKROVERKAPSELUNG UND IHRE VERWENDUNGEN
MICROENCAPSULATION SYSTEMS AND APPLICATIONS OF SAME

(30) Priorité: 20.01.2003 FR 0300578
(43) Date de publication de la demande: 02.11.2005
(73) Titulaire: CENTRE NATIONAL DE LA RECHERCHE SCIENTIFIQUE (CNRS), 75794 Paris Cedex 16 (FR); Université Paris XI, 91405 Orsay Cedex (FR)
(72) Inventeur: BOCHOT, Amélie, F-75014 Paris (FR); ALPHANDARY, Huguette, F-92170 Vanves (FR); DUCHENE, Dominique, F-75116 Paris (FR); FATTAL, Elias, F-75012 Paris (FR)
(74) Mandataire: Novagraaf Technologies
(86) Numéro de dépôt international: PCT/FR2004/000119
(87) Numéro de publication internationale: WO 2004/066906

(56) Documents cités:
- EP-A- 1 159 882
- US-A- 3 202 731

## Description

L'invention a pour objet des systèmes pour l'encapsulation de substances d'intérêt et leurs applications.

La microencapsulation regroupe l'ensemble des technologies qui permettent la préparation de microbilles individualisées constituées d'un matériau enrobant contenant une matière active. Les microbilles, encore appelées microparticules, présentent une taille comprise entre 1 µm et plusieurs mm et contiennent typiquement entre 5 et 90 % (en poids) de matière active. Les matières actives sont d'origine très variée : principes actifs pharmaceutiques, actifs cosmétiques, additifs alimentaires, produits phytosanitaires, essences parfumées, microorganismes, cellules ou encore catalyseurs de réactions chimiques. Les matériaux enrobants sont des polymères d'origine naturelle ou synthétique, hydrophobes ou hydrophiles, ou des lipides.

Les microbilles préparées à partir de matériaux polymères hydrophobes sont généralement préparées par des techniques de séparation de phase (coacervation ou extraction-évaporation de solvant) ou par polymérisation ou polycondensation. Les techniques de séparation de phase utilisent généralement des solvants organiques qui présentent un certain nombre d'inconvénients : élimination dans l'atmosphère, rémanence au sein des systèmes galéniques, dénaturation de certaines molécules microencapsulées. Les méthodes par polymérisation ou polycondensation, si elles possèdent l'avantage de ne pas utiliser de solvant, présentent l'inconvénient d'utiliser des matériaux très réactifs capables de réagir avec les substances encapsulées au sein des microbilles. Enfin, la plupart des matériaux composant ces matières premières sont des substances synthétiques dont la nocivité pour l'environnement ou l'organisme n'est pas toujours connue.

Les microbilles formées à partir de matériaux polymères hydrophiles sont généralement préparées par des techniques de gélification ou de coacervation. Cette technique qui permet d'encapsuler des molécules sous forme liquide ou solide est basée sur la désolvatation de macromolécules conduisant à une séparation de phases au sein d'une solution. En général, avec les polymères hydrophiles, on procède à une coacervation complexe où la désolvatation s'effectue sur deux polymères. Elle peut s'effectuer par exemple par ajustement du pH de la solution contenant les polymères de manière à ce que les charges positives du premier polymère équilibrent les charges négatives du second formant une précipitation et un enrobage des matériaux à encapsuler. La membrane gélifiée est ensuite réticulée par du glutaraldéhyde. Cette technique s'adresse surtout à des matériaux lipophiles (huiles végétales ou minérales, huiles essentielles). Les microbilles peuvent être préparées par gélification ionique. Dans ce cas, une solution d'alginate ou de pectinate de sodium est injectée (par prilling) dans une solution de chlorure de calcium. Au contact de cette solution, les gouttes gélifient formant des microbilles.

En ce qui concerne l'utilisation de matériaux lipidiques, la microencapsulation s'effectue par gélification thermique. Ce procédé appelé "hot melt" repose sur la fusion du matériau d'enrobage. La matière active à encapsuler est dissoute ou dispersée dans ce matériau fondu. L'ensemble est émulsionné dans une phase dispersante dont la température est maintenue supérieure à la température de fusion de l'enrobage. La solidification des globules dispersés est obtenue en refroidissant brutalement le milieu.

A côté de ce type de microencapsulation particulaire, on distingue les phases molles (micelles, liposomes, sphérulites, microémulsions, émulsions...) et l'encapsulation moléculaire (cyclodextrines).

Les travaux des inventeurs dans ce domaine ont montré qu'il était possible de former de nouveaux systèmes utilisables pour piéger des substances d'intérêt par simple agitation orbitale, à température ambiante ou voisine de l'ambiante, à partir de composés capables d'interagir avec des substances huileuses.

L'invention a donc pour but de fournir de nouveaux systèmes pour la microencapsulation de grande stabilité au stockage, possédant notamment une sensibilité élevée au cisaillement, ce qui permet de libérer facilement leur contenu.

L'invention a également pour objet les applications de ces systèmes, en particulier en thérapeutique, en cosmétique et dans le domaine alimentaire.

Les systèmes pour microencapsulation de l'invention sont caractérisés en ce qu'ils sont élaborés à partir de substances huileuses et de sucres, et forment un ensemble essentiellement organisé.

Cette organisation correspond plus particulièrement à des empilements de structures cristallines. Des systèmes de ce type présentent par exemple une organisation en structures cristallines de type hexagonal ou pseudo-hexagonal.

Le terme « sucre », tel qu'utilisé dans la description et les revendications, désigne des poly-et/ou des oligosaccharides, et/ou des amidons, et/ou leurs dérivés.

Dans un mode préféré de réalisation de l'invention, lesdits sucres sont des oligosaccharides et, en particulier, des cyclodextrines et leurs dérivés.

La cyclodextrine α est particulièrement avantageuse compte tenu de son aptitude à former des complexes d'inclusion avec des substances huileuses.

Dans d'autres modes de réalisation de l'invention, lesdits sucres sont des polysaccharides, comme l'amidon.

Les différents sucres et substances huileuses ci-dessus correspondent à des molécules naturelles ou synthétiques.

Les substances huileuses entrant dans la composition des systèmes de l'invention sont liquides ou semi-solides et sont capables de former la phase huileuse d'une émulsion. On citera plus spécialement les huiles ou leurs composants. Il s'agit notamment d'acides gras, de mono-, di- ou triglycérides.

Des huiles appropriées comprennent des huiles végétales, comme l'huile de soja, de germe de blé, d'avocat ou d'amande douce, ou des huiles animales, comme l'huile d'onagre, des huiles synthétiques ou minérales comme l'huile de paraffine.

Dans les systèmes définis ci-dessus, les substances huileuses peuvent être à l'état dispersé et/ou sous forme de complexes d'inclusion, par exemple avec les cyclodextrines et, en particulier, la cyclodextrine α.

Des substances d'intérêt peuvent être piégées dans lesdites substances huileuses.

L'invention vise donc les systèmes renfermant, en outre, une ou plusieurs substances d'intérêt choisies parmi des substances n'affectant pas l'organisation de l'ensemble et sa stabilité.

Ces substances d'intérêt sont des substances hydrosolubles ou des substances liposolubles.

L'invention permet avantageusement de formuler des molécules fragiles, sensibles à l'oxydation ou à la lumière, ou pouvant être dénaturées par les méthodes d'encapsulation classiques, qui font appel à des solvants organiques, et/ou des tensio-actifs, dont l'extraction totale est difficile, voire impossible, à une température élevée, ou encore à des cisaillements trop importants.

Selon un mode de réalisation de l'invention, les systèmes de l'invention se présentent notamment sous forme de billes solides à structure pleine. De telles billes présentent, de manière générale, une granulométrie du micron à plusieurs centimètres, notamment de 0, 1 à 8 mm, ou encore de 0,1 à 5 mm, en particulier de 0,5 à 3 mm.

Dans un autre mode de réalisation de l'invention, les systèmes se présentent sous forme de phases compacte ou fluide.

Ces différents systèmes peuvent être également séchés, lyophilisés, mis en suspension dans un milieu aqueux ou non aqueux, liquide ou gélifié.

Sous forme de billes séchées, lyophilisées ou non, les systèmes de l'invention peuvent être introduits dans des gélules.

L'invention vise également un procédé de préparation des systèmes définis ci-dessus.

Ce procédé est caractérisé en ce qu'il comprend les étapes
- d'addition de substances huileuses à une solution ou suspension aqueuse d'un sucre capable d'interagir avec lesdites substances huileuses en formant les systèmes de l'invention ;
- d'agitation modérée du milieu, à une température de 15 à 40 ° C, de préférence de 18 à 37 ° C, plus particulièrement de 20 à 30° C, notamment de 20 à 25°C, et de récupération des systèmes formés.

L'agitation est réalisée dans des conditions de vitesse et de durée permettant d'obtenir des billes solides de structure pleine, ces dernières étant récupérées, lavées et éventuellement séchées ou lyophilisées. En variante, l'agitation est arrêtée avant la formation de ces billes, et les phases intermédiaires sont récupérées, plus spécialement la phase compacte définie plus haut.

Pour améliorer la solubilité des molécules d'intérêt, on peut prévoir d'utiliser un co-solvant.

De manière avantageuse, ce procédé ne fait appel ni à l'utilisation de solvants organiques, ni à une étape de chauffage, ni à une consommation importante d'énergie, ce qui constitue une avancée de grand intérêt dans le domaine de l'encapsulation.

On notera que ce procédé de fabrication des billes ne nécessite pas d'appareillages spéciaux pour la fabrication, tels que des turbines, des homogénéiseurs, des hottes spécifiques. L'agitation requise pour former les billes ne consomme que très peu d'énergie. Le procédé de fabrication ne fait pas intervenir de solvants organiques ni de tensio-actifs, ce qui représente un avantage en terme de sécurité. Les matériaux mis en jeu pour la formation des billes et des phases intermédiaires sont non toxiques et biodégradables (substances huileuses, sucres). Il est possible de former des billes avec ces sucres, notamment les poly et oligosaccharides, et en particulier les cyclodextrines sans réticulation. Les matériaux utilisés sont disponibles facilement sur le marché et à un coût modéré.

L'invention fournit ainsi des moyens de grande simplicité et de faible coût pour fabriquer de nouveaux systèmes utilisables dans de nombreux secteurs de l'industrie.

L'invention vise en particulier leur application en thérapeutique où ils permettent notamment d'encapsuler des principes actifs de médicaments et constituent de nouvelles formes galéniques ou toute forme intermédiaire utilisables dans la réalisation d'autres formes d'administration (gélules, granules, compacts...) pour une administration par voie orale. Les principes actifs encapsulés selon l'invention peuvent être également administrés par voie cutanée et sur les muqueuses.

L'invention vise également notamment leur application en cosmétique pour l'encapsulation de substances actives en cosmétologie et/ou de pigments et/ou de colorants et/ou des produits naturels ou synthétiques entrant dans la composition de parfums, arômes, fragrances. L'utilisation des systèmes permet ainsi de réaliser de nouvelles formulations, utilisables, par exemple, comme produits de maquillage. Des formes et présentations telles que des compacts, sticks de billes, gels fluides de billes, billes de bain ou autres peuvent être ainsi élaborées.

Une autre application d'intérêt concerne le domaine alimentaire. Des nouvelles formulations de produits diététiques, aliments ou alicaments peuvent être préparées.

On notera que, dans ces applications, les systèmes présentent l'avantage de masquer les odeurs ou les goûts désagréables.

On citera encore l'application des systèmes de l'invention dans les industries agronomiques, par exemple pour l'encapsulation de pesticides, ou des peintures contenant des pigments minéraux ou organiques utilisant différents types de liants (à l'eau, à l'huile...) sous forme liquide ou de pâte, peinture à l'état sec (crayons, pastels, poudre particulée...) enduits gras.

D'autres caractéristiques et avantages de l'invention seront donnés dans les exemples qui suivent qui se rapportent à des modes de réalisation de l'invention mettant en jeu, à titre illustratif, la cyclodextrine α, en tant qu'oligosaccharide, et des huiles végétales ou animales, comme substances huileuses.

Dans ces exemples, il sera fait référence aux figures 1 à 5, qui représentent, respectivement,
- les figures 1a à 1c : des photos en microscopie électronique à balayage sur des billes entières avant lyophilisation (figure 1a), lyophilisées (figure 1b) : (Gx30) et sur leur surface (Gx625) (Figure 1c) ;
- les figures 2a et 2b : des photos en microscopie électronique à transmission d'une cryofracture de billes (Gx30000) (figure 2a) ; la partie zoomée (Gx78000) (figure 2b) ;
- les figures 3a à 3c : une photo de cristaux observés en microscopie optique (Gx650) (Figure 3a) ; une photo en microscopie optique confocale de coupes semi-fines de billes marquées au Rouge Nil incluses en résine, image en transmission (Gx64) (figure 3b), et une photo en microscopie électronique à balayage de cristaux après extraction à l'isopentane (Gx4000) (figure 3c).

### Exemple 1 : formation de billes à partir de cyclodextrine α et d'huiles végétales.

Dans une première étape, on introduit dans un flacon de la cyclodextrine (α-CD) (3 à 6 % m/m) solubilisée ou non dans une phase aqueuse représentant 67 à 82 % de la masse totale. Une phase huileuse formée d'huile de soja (15 à 30 % m/m) est ajoutée à la surface de l'eau. Le pH de la phase aqueuse peut être ajusté de pH 2 à 9,3. La molécule à encapsuler peut être additionnée à l'une des deux phases : une molécule hydrosoluble peut être ajoutée dans la phase aqueuse et une molécule liposoluble peut être ajoutée à la phase huileuse. Le flacon est alors bouché, puis mis sous agitation (ROTATEST, Bioblock Scientific) à une vitesse de 200 tours/minute, dans un bain-marie thermostaté (28°C). Après un délai d'environ 0,5 à 30 jours, mais plus généralement de 2 à 3 jours, des billes de couleur blanche plus ou moins sphériques se forment. Plusieurs états intermédiaires (états fluide, puis compact) précèdent la formation des billes. La cinétique de formation des billes, dans les conditions testées, est plus lente aux pH acides. Aux pH de 9,5 à 10,3, les phases restent compactes.

En opérant avec des concentrations d'huile de soja de 12-24% m/m, d'eau osmosée de 70-82% m/m et de CD-α de 3,3-6%m/m, on obtient, en 0,5 à 5 jours, des billes de 0,5 à 3 mm, et un milieu de suspension clair ne présentant pas ou peu de globules huileux.

Pour les essais ci-après, un mélange ternaire de 2,88 ml d'huile de soja, 10 ml d'eau osmosée de pH 5,5-6, et 0,813 g d'α-cyclodextrine a été utilisé.

Les billes obtenues sont stables (pendant au moins 3 ans) et en suspension dans une phase dispersante dont la turbidité varie. En effet, les billes préparées dans les conditions ci-dessus présentent une distribution de taille homogène et se retrouvent dans une phase dispersante claire. Les billes qui présentent une distribution de taille plus hétérogène, se retrouvent dans une phase blanchâtre.

Les billes en suspension dans l'eau, séchées ou lyophilisées, peuvent être dispersées au sein d'hydrogels, par exemple de Carbomer, de cellulose ou de poloxamère 407.

On notera que ces traitements, notamment leur séchage ou leur lyophilisation n'altèrent pas leurs caractéristiques, ce qui présente un intérêt pour leur conservation.

Les billes sont capables de subir d'autres opérations telles que la filtration à pression normale, la centrifugation à faible vitesse, le séchage à l'étuve (les billes deviennent alors transparentes).

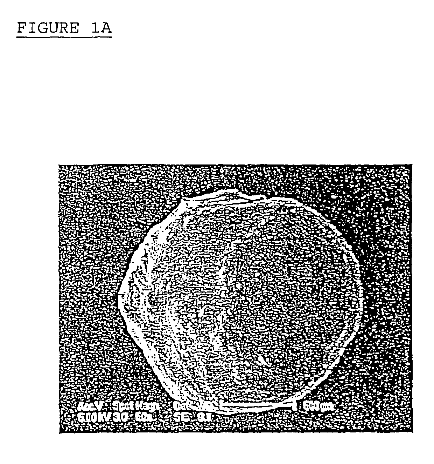

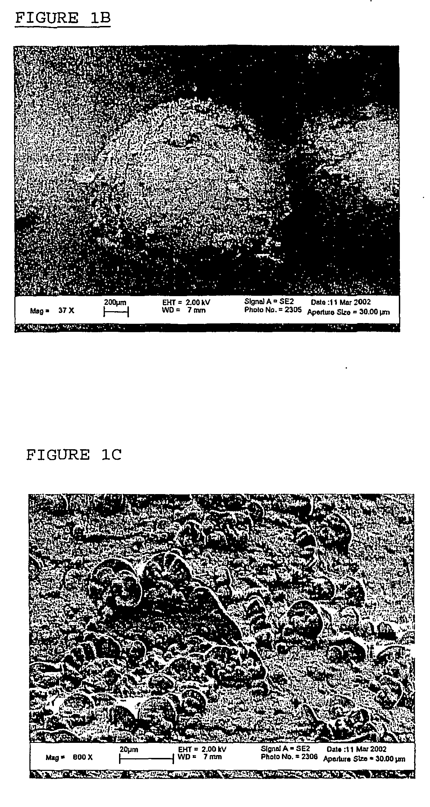

On rapporte sur les figures 1a-1c, les photos en microscopie électronique à balayage de la surface d'une bille selon l'invention avant lyophilisation (figure 1a), d'une bille lyophilisée (figure 1b), (Gx30), et une vue de la surface (Gx625) (figure 1c). Cet examen montre une surface avec des aspérités, que les billes soient ou non lyophilisées.

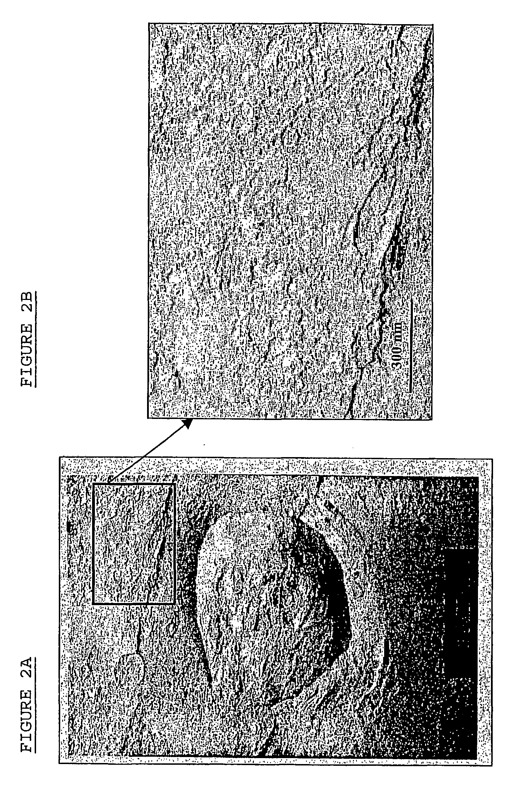

La structure interne des billes a également été étudiée. A cet effet, les billes en suspension dans l'eau ont subi une cryofracture et les répliques ont été observées en microscopie électronique à transmission. Comme le montrent les figures 2a et 2b, les billes ont une structure matricielle, c'est-à-dire pleine, présentant des structures globuleuses et des éléments réguliers, anguleux de 30 nm environ.

Les billes sont constituées de compartiments lipophiles (huile) et hydrophiles (cyclodextrine). Les images obtenues en microscopie confocale montrent une répartition de la calcéine (marqueur fluorescent hydrophile) à la surface des billes et une répartition sporadique du Rouge Nil (marqueur fluorescent des lipides) à la surface et à l'intérieur de ces dernières. L'analyse microscopique des milieux de suspension des billes ne met pas en évidence de présence importante de gouttelettes d'huile, montrant que l'huile est bien piégée dans le système.

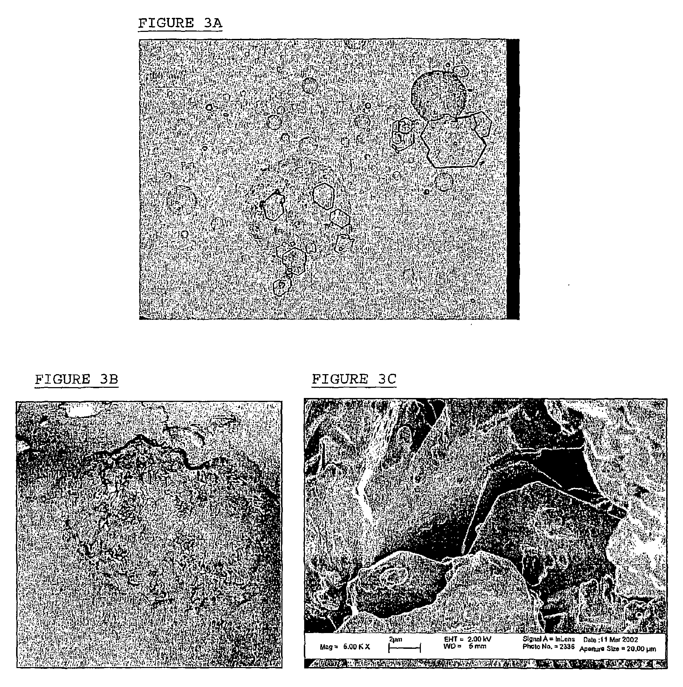

La présence de nombreux cristaux pseudohexagonaux, de taille hétérogène allant de 10 nm à quelques microns au sein des billes a été montrée par microscopie optique (Figure 3a), confocale (figure 3b) et électronique à balayage (figure 3c). Ces cristaux ont pu être isolés par traitement à l'isopentane et mis en évidence par microscopie électronique à balayage, microscopie électronique à transmision (cryofracture, coloration négative, coupes ultrafines, diffraction des électrons) et par diffraction des rayons X aux petits et aux grands angles.

### Stabilité des billes dans les milieux biologiques

Dans l'optique d'une administration des billes par voie orale de principes actifs, des essais de stabilité des billes lyophilisées et non lyophilisées ont été réalisés dans des milieux simulant les liquides digestifs mis sous agitation à 37°C (estomac milieu pH 1,2 ; intestins pH 6,8 : milieux décrits par la Pharmacopée Américaine USP XXIII).

Les billes sont stables environ 5h30 dans le milieu stomacal et environ4h30 dans le milieu intestinal. Au-delà de ces temps, une diminution du nombre de billes et de leur taille est observée. Des résultats quasi-similaires ont été enregistrés avec les billes lyophilisées et non lyophilisées.

### Exemple 2 : Encapsulation de molécules dans les billes

On opère comme décrit dans l'exemple 1, mais en utilisant comme molécules d'intérêt, des molécules actives en thérapeutique ou utilisables en cosmétique comme des pigments ou des colorants, de l'acétate de vitamine E, de la benzophénone, de l'isotrétinoïne.

On rapporte dans le tableau suivant le diamètre des billes obtenues et le temps de formation

| Molécules | Concentration | Diamètre des billes | Temps de formation |
|---|---|---|---|
| Pharmacie 5-Méthoxypsoralène | 0.52mg/ml huile | 2 mm | 2 jours |
| Cosmétique Acétate de vitamine E | 23.4 mg/ml huile | 2 mm | 3 jours |
| Acétate de vitamine E | 46.9 mg/ml huile | 2 mm | 4 jours |
| Vitamine E | 23.4 mg/ml huile | 2 mm | 7 jours |
| Benzophénone | 1.9 mg/ml huile | 2 mm | 3 jours |
| Marqueur Fluorescent | | | |
| Calcéine | 0.3 mg/ml eau | 2 mm | 7 jours |
| Rouge Nil | | 2 mm | 3 jours |
| Colorants liposolubles oxyde de chrome (vert) | | | 3 jours |
| Jaune de méthyle | 5.1 mg /ml huile | | 4 jours |
| Sel de cobalt (bleu) | | | 3 jours |
| Mica, dioxyde de | | | |
| titane, oxyde de fer | | | 5 jours |
| Colorants hydrosolubles | | | |
| Bleu de méthylène | | | 4 jours |
| Divers Cacao | 2.7 mg/ml huile | 2.5 mm | 7 jours |

On constate que la présence des molécules lipophiles ou hydrophiles testées ne modifie pas les caractéristiques des billes, que ce soit leur taille ou leur temps de formation. De plus, il a été montré que 30% de l'acétate de vitamine E est encapsulé (détermination par HPLC).

Des billes renfermant des parfums, par exemple Femme® de ROCHAS, ont été également préparées.

### Exemple 3: Formation des billes en présence de co-solvant

On opère comme décrit dans l'exemple 1, mais en ajoutant dans l'huile ou dans l'eau un co-solvant.

| Co-solvant | Diamètre des billes | Temps de formation |
|---|---|---|
| Ethanol (200 microl dans 2.68 ml d'huile) | 1mm | 9 jours |
| Miglyol 810 (200 microl dans 2.68 ml d'huile) | 1mm | 3 jours |
| Glycérine 5% dans l'eau osmosée | 3 mm | 3 jours |
| Glycérine 10% dans l'eau osmosée | 2 mm | 3 jours |
| Glycérine 15% dans l'eau osmosée | 1.5 mm | 3 jours |

### Exemple 4 : Formation des billes après pré-émulsification de la phase aqueuse avec la phase huileuse

On opère comme décrit dans l'exemple 1 mais la phase aqueuse contenant la cyclodextrine (α) est émulsionnée avec la phase huileuse à l'aide d'une turbine d'agitation. Le mélange obtenu est soumis alors aux conditions d'agitation décrites dans l'exemple 1.

## Revendications

1. Systèmes pour microencapsulation, **caractérisés en ce qu'**ils sont élaborés à partir de substances huileuses et de sucres, et forment un ensemble essentiellement organisé correspondant à des empilements de structures cristallines.

2. Systèmes selon la revendication 1, **caractérisés en ce qu'**ils présentent une organisation en structures cristallines de type hexagonal ou pseudo-hexagonal.

3. Systèmes selon la revendication 1 ou 2, **caractérisés en ce que** les sucres sont des poly-et/ou des oligosaccharides, et/ou des amidons, et/ou leurs dérivés.

4. Systèmes selon la revendication 3, **caractérisés en ce que** les oligosaccharides sont des cyclodextrines.

5. Systèmes selon la revendication 4, **caractérisés en ce qu'**il s'agit de cyclodextrine α.

6. Systèmes selon l'une quelconque des revendications précédentes, **caractérisés en ce que** les substances huileuses sont des acides gras, des mono-, di- ou triglycérides.

7. Systèmes selon la revendication 6, **caractérisés en ce que** les substances huileuses sont des huiles végétales, comme l'huile de soja, de germe de blé, d'avocat ou d'amande douce, des huiles animales, comme l'huile d'onagre, ou des huiles synthétiques ou minérales.

8. Systèmes selon l'une quelconque des revendications précédentes, **caractérisés en ce que** lesdites substances huileuses sont à l'état dispersé et/ou sous forme de complexes d'inclusion, par exemple avec les cyclodextrines et, en particulier, la cyclodextrine α.

9. Systèmes selon l'une quelconque des revendications précédentes, **caractérisés en ce qu'**ils renferment, en outre, une ou plusieurs substances d'intérêt.

10. Systèmes selon la revendication 9, **caractérisés en ce qu'**il s'agit de substances hydrosolubles ou de substances liposolubles.

11. Systèmes selon la revendication 10, **caractérisés en ce que** la ou lesdites substances sont thérapeutiquement actives, en particulier à faible dose.

12. Systèmes selon la revendication 10, **caractérisés en ce que** la ou lesdites substances sont utilisables en cosmétique.

13. Systèmes selon l'une quelconque des revendications précédentes, **caractérisés en ce qu'**ils se présentent sous forme de billes solides à structure pleine.

14. Systèmes selon la revendication 13, **caractérisés par** une granulométrie du micron à plusieurs centimètres, notamment de 0,1 à 8 mm, ou encore de 0,1 à 5 mm, en particulier de 0,5 à 3 mm.

15. Systèmes selon l'une quelconque des revendications 1 à 14, **caractérisés en ce qu'**ils se présentent sous forme de phases compacte.

16. Systèmes selon l'une quelconque des revendications précédentes, **caractérisés en ce qu'**ils se présentent sous forme de billes en suspension, de billes séchées ou lyophilisées, redispersées ou non dans un liquide aqueux ou non aqueux ou dans un gel.

17. Procédé de préparation de systèmes selon l'une quelconque des revendications précédentes, **caractérisé en ce qu'**il comprend les étapes
- d'addition de substances huileuses à une solution ou suspension aqueuse de sucre capable d'interagir avec lesdites substances huileuses en formant des systèmes essentiellement organisés correspondant à des empilements de structures cristallines notamment de type hexagonal ou pseudo-hexagonal ;
- d'agitation modérée du milieu, à une température de 15 à 40°C, de préférence de 18 à 37°C, plus particulièrement de 20 à 30°C, notamment de 20 à 25°C, et de récupération des systèmes formés.

18. Procédé selon la revendication 17, **caractérisé en ce que** l'agitation est réalisée dans des conditions de vitesse et de durée permettant d'obtenir des billes solides de structure pleine, ou une phase compacte ou fluide.

## Claims

1. Microencapsulation systems, **characterized in that** they are developed from oily substances and from carbohydrates, and form an essentially organized assembly corresponding to stacks of crystalline structures.

2. The systems according to claim 1, **characterized in that** they present an organization in the form of hexagonal- or pseudohexagonal-type crystalline structures.

3. The systems according to claim 1 or 2, **characterized in that** the carbohydrates are polysaccharides and/or oligosaccharides, and/or starches, and/or derivatives thereof.

4. The systems according to claim 3, **characterized in that** the oligosaccharides are cyclodextrins.

5. The systems according to claim 4, **characterized in that** it is α-cyclodextrin.

6. The systems according to any of the preceding claims, **characterized in that** the oily substances are fatty acids, monoglycerides, diglycerides or triglycerides.

7. The systems according to claim 6, **characterized in that** the oily substances are plant oils, such as soya oil, wheatgerm oil, avocado oil or sweet almond oil, animal oils, such as onager oil, or synthetic oils or mineral oils.

8. The systems according to any of the preceding claims, **characterized in that** said oily substances are in the dispersed state and/or in the form of inclusion complexes, for example with cyclodextrins, and in particular, α-cyclodextrin.

9. The systems according to any of the preceding claims, **characterized in that** it contains, in addition, one or several substances of interest.

10. The systems according to claim 9, **characterized in that** they are water-soluble substances or liposoluble substances.

11. The systems according to claim 10, **characterized in that** said substance(s) are therapeutically active, in particular at low dose.

12. The systems according to claim 10, **characterized in that** said substance(s) can be used in cosmetic.

13. The systems according to any of the preceding claims, **characterized in that** they come in the form of solid beads with a dense structure.

14. The systems according to claim 13, **characterized by** a grading from one micron to several centimeters, in particular from 0,1 to 8 mm, or else from 0,1 to 5 mm, in particular of 0,5 to 3 mm.

15. The systems according to any of claims 1 to 14, **characterized in that** they come in the form of compact phases.

16. The systems according to any of the preceding claims, **characterized in that** they come in the form of beads in suspension, or of dried or lyophilized beads, redispersed or not in an aqueous or nonaqueous liquid or in a gel.

17. A method for preparing the systems according to any of the preceding claims, **characterized in that** it comprises the steps of:
- addition of oily substances to an aqueous solution or suspension of carbohydrates capable of interacting with said oily substances by forming essentially organized systems corresponding to stacks of crystalline structures, in particular hexagonal- or pseudohexagonal-type structures;
- moderate agitation of the medium, at a temperature of 15 to 40°C, preferably of 18 to 37°C, more particularly of 20 to 30°C, especially of 20 to 25°C, and recovery of the systems formed.

18. The method according to claim 17, **characterized in that** the agitation is carried out under conditions of speed and of duration allowing to obtain solid beads with a dense structure, or a compact or fluid phase.

## Patentansprüche

1. Systeme zur Mikroverkapselung, **dadurch gekennzeichnet: dass** sie aus öligen Substanzen und Kohlenstoffen hergestellt werden, und ein größtenteils organisiertes Gebilde darstellen, das aufeinander gestapelten kristallinen Strukturen entspricht.

2. Systeme nach Anspruch 1, **dadurch gekennzeichnet, dass** sie eine Organisation in hexagonalen oder pseudo-hexagonalen kristallinen Strukturen aufweisen.

3. Systeme nach Anspruch 1 oder 2, **dadurch gekennzeichnet, dass** die Kohlenhydrate Polysaccharide und/oder Oligosaccharide sind und/oder Stärke, und/oder Derivate hiervon.

4. Systeme nach Anspruch 3, **dadurch gekennzeichnet, dass** die Oligosaccharide Cyclodextrine sind.

5. Systeme nach Anspruch 4, **dadurch gekennzeichnet, dass** es sich um α-Cyclodextrin handelt.

6. Systeme nach einem beliebigen der vorgenannten Ansprüche, **dadurch gekennzeichnet, dass** die öligen Substanzen Fettsäuren, Monoglyzeride, Diglyzeride oder Triglyzeride sind.

7. Systeme nach Anspruch 6, **dadurch gekennzeichnet, dass** die öligen Substanzen Pflanzenöle wie Soja-, Weizenkeim-, Avocado-, oder Süßmandelöl, tierische Öle wie Onageröl, oder synthetische oder Mineralöle sind.

8. Systeme nach einem beliebigen der vorgenannten Ansprüche, **dadurch gekennzeichnet, dass** die besagten Öle dispersiert und/oder in Form von Inklusionskomplexen vorliegen, zum Beispiel mit Cyclodextrinen und insbesondere mit α-Cyclodextrin.

9. Systeme nach einem beliebigen der vorgenannten Ansprüche, **dadurch gekennzeichnet, dass** sie zusätzlich eine oder mehrere Substanzen von Interesse enthalten.

10. Systeme nach Anspruch 9, **dadurch gekennzeichnet, dass** es sich um wasser- oder fettlösliche Substanzen handelt.

11. Systeme nach Anspruch 10, **dadurch gekennzeichnet, dass** besagte Substanz(en) therapeutisch aktiv sind, insbesondere bei niedriger Dosierung.

12. Systeme nach Anspruch 10, **dadurch gekennzeichnet, dass** besagte Substanz(en) für Kosmetik verwendet werden können.

13. Systeme nach einem beliebigen der vorgenannten Ansprüche, **dadurch gekennzeichnet, dass** sie in Form von vollen festen Kügelchen vorliegen.

14. Systeme nach Anspruch 13, **dadurch gekennzeichnet, dass** die Korngrößenverteilung zwischen einem Mikron und mehreren Zentimetern liegt, insbesondere zwischen 0,1 und 8 mm, oder auch zwischen 0,1 und 5 mm, insbesondere zwischen 0,5 und 3 mm.

15. Systeme nach einem beliebigen der vorgenannten Ansprüche 1 bis 14, **dadurch gekennzeichnet, dass** sie in kompakten Phasen vorliegen.

16. Systeme nach einem beliebigen der vorgenannten Ansprüche, **dadurch gekennzeichnet dass** die Kügelchen schwebend, getrocknet oder gefriergetrocknet, eventuell wieder dispersiert in einer wässrigen oder nicht wässrigen Lösung oder in einem Gel vorliegen.

17. Ein Verfahren zur Herstellung der Systeme nach einem beliebigen der vorgenannten Ansprüche, **dadurch gekennzeichnet, dass** es folgende Schritte enthält:
- das Zufügen öliger Substanzen zu einer wässrigen Lösung oder Suspension aus Kohlehydraten, die mit den besagten öligen Substanzen interagieren kann, indem größtenteils organisierte Systeme gebildet werden, die aufeinander gestapelten, insbesondere hexagonalen oder pseudo-hexagonalen, kristallinen Strukturen entsprechen;
- moderates Schütteln des Mediums bei Temperaturen von 15° bis 40°C, vorzugsweise bei 18° bis 37°C, und insbesondere bei 20° bis 30°C, vor allem bei 20° bis 25°C, und Rückgewinnung der gebildeten Systeme.

18. Verfahren nach Anspruch 17, **dadurch gekennzeichnet, dass** das Schütteln bei einer Geschwindigkeit und einer Dauer durchgeführt wird, bei der feste Kügelchen mit voller Struktur oder eine kompakte oder flüssige Phase erhalten werden können.
